(19) Europäisches Patentamt
European Patent Office
Office européen des brevets

(11) **EP 2 749 208 B2**

(12) **NEW EUROPEAN PATENT SPECIFICATION**
After opposition procedure

(45) Date of publication and mention
of the opposition decision:
**03.08.2022   Bulletin 2022/31**

(45) Mention of the grant of the patent:
**24.04.2019   Bulletin 2019/17**

(21) Application number: **13195412.5**

(22) Date of filing: **03.12.2013**

(51) International Patent Classification (IPC):
*A61B 8/14* (2006.01)        *A61B 8/00* (2006.01)
*G06T 5/00* (2006.01)        *G06T 5/50* (2006.01)
*A61B 5/00* (2006.01)

(52) Cooperative Patent Classification (CPC):
**A61B 5/7425; A61B 5/0035; A61B 5/0095;
A61B 5/7435; A61B 8/4272; G06T 5/008;
G06T 5/50;** A61B 5/748; G06T 2207/10132;
G06T 2207/20104; G06T 2207/20221;
G06T 2207/30104

(54) **Object information acquiring apparatus**

Objektinformationserfassungsvorrichtung und Objektinformationserfassungsverfahren

Appareil d'acquisition d'informations d'objets et procédé d'acquisition d'informations d'objets

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **28.12.2012   JP 2012286546**

(43) Date of publication of application:
**02.07.2014   Bulletin 2014/27**

(73) Proprietor: **Canon Kabushiki Kaisha
Tokyo 146-8501 (JP)**

(72) Inventors:
• **Nakamura, Shuichi
Tokyo, Tokyo 146-8501 (JP)**
• **Abe, Hiroshi
Tokyo, Tokyo 146-8501 (JP)**

(74) Representative: **TBK
Bavariaring 4-6
80336 München (DE)**

(56) References cited:
EP-A1- 1 493 380        EP-B1- 0 646 263
WO-A2-2012/014390        WO-A2-2013/056089
JP-A- 2012 075 464        US-A- 5 042 077
US-A- 5 797 397        US-A1- 2003 053 668
US-A1- 2004 220 465        US-A1- 2010 049 044
US-A1- 2010 179 413

• **RONALD E KUMON ET AL: "Frequency-Domain
Analysis of Photoacoustic Imaging Data From
Prostate Adenocarcinoma Tumors in a Murine
Model", ULTRASOUND IN MEDICINE AND
BIOLOGY, NEW YORK, NY, US, vol. 37, no. 5, 20
January 2011 (2011-01-20), pages 834-839,
XP028192411, ISSN: 0301-5629, DOI:
10.1016/J.ULTRASMEDBIO.2011.01.012
[retrieved on 2011-01-28]**
• **JAEGER M ET AL: "Improved contrast deep
optoacoustic imaging using
displacement-compensated averaging: breast
tumour phantom studies;Improved contrast deep
optoacoustic imaging of breast phantoms",
PHYSICS IN MEDICINE AND BIOLOGY,
INSTITUTE OF PHYSICS PUBLISHING, BRISTOL
GB, vol. 56, no. 18, 18 August 2011 (2011-08-18),
pages 5889-5901, XP020210558, ISSN: 0031-9155,
DOI: 10.1088/0031-9155/56/18/008**
• **DE LA ZERDA A ET AL: "Ultrahigh sensitivity
carbon nanotube agents for photoacoustic
molecular imaging in living mice", NANO
LETTERS, AMERICAN CHEMICAL SOCIETY, vol.
10, no. 6, 9 June 2010 (2010-06-09), pages
2168-2172, XP002690836, ISSN: 1530-6984, DOI:
10.1021/NL100890D [retrieved on 2010-05-25]**
• **SONG HU et al.: "Label-free photoacoustic
ophthalmic angiography", Optics Letters, vol. 35,
no. 1, 1 January 2010 (2010-01-01), pages 1-3, DOI:
10.1364/OL.35.000001**
• **XUEDING WANG et al.: "Noninvasive imaging of
hemoglobin concentration and oxygenation in
the rat brain using high-resolution photoacoustic
tomography", Journal of Biomedical Optics, vol.
11, no. 2 March 2006 (2006-03), page 024015,
[retrieved on 2006-05-03]**

EP 2 749 208 B2

**Description**

BACKGROUND OF THE INVENTION

Field of the Invention

The present invention relates to technology of displaying image data in an object information acquiring apparatus.

Description of the Related Art

Various proposals have been previously made in relation to the technology of imaging a tomographic image of an object using light. Among such proposals, there is a photoacoustic tomographic image imaging apparatus (hereinafter referred to as "photoacoustic imaging apparatus") which uses the photoacoustic tomography (PAT) technology.

A photoacoustic imaging apparatus emits measuring light such as a pulsed laser beam to an object, receives acoustic waves that are generated when the measuring light is absorbed by the living body tissues in the object, and performs analytical processing to the acoustic waves so as to visualize information (function information) related to the optical characteristics inside the living body.

Large amounts of oxygenated hemoglobin contained in arterial blood and large amounts of reduced hemoglobin contained in venous blood absorb the laser beam and generate acoustic waves, but the absorptivity of the laser beam differs depending on the wavelength. For example, oxygenated hemoglobin has a high rate of absorbing light of 805 nm or less, and reduced hemoglobin has a high rate of absorbing light of 805 nm or more.

Thus, by emitting laser beams of different wavelengths and measuring the respective acoustic waves, it is possible to visualize the distribution status of oxygenated hemoglobin and reduced hemoglobin, and calculate the amount of hemoglobin or oxygen saturation by analyzing the obtained information. Since this kind of function information can be used as the information related to vascularization near the tumor cells, the photoacoustic imaging apparatus is known to be particularly effective for the diagnosis of skin cancer and breast cancer.

Meanwhile, an ultrasonic imaging apparatus is also known as an image diagnosing apparatus which can perform imaging without exposure and noninvasively as with a photoacoustic imaging apparatus. An ultrasonic imaging apparatus emits ultrasonic waves to a living body, and receives acoustics waves which are generated as a result of the ultrasonic waves that propagated within the object being reflected off the tissue interface, which has different acoustic characteristics (acoustic impedance) in the living body tissues. In addition, by performing analytical processing to the received acoustic waves, information (shape information) related to the acoustic characteristics inside the living body, which is the object, is visualized. The visualized shape information is unique in that it can offer an indication of the shape of the living body tissues.

While a photoacoustic imaging apparatus can acquire function information, with only the function information, it is difficult to determine from which part of the living body tissues such function information was generated. Thus, proposed is technology of incorporating an ultrasonic imaging unit inside a photoacoustic imaging apparatus, and simultaneously acquiring shape information. For example, Japanese Patent Application Publication No. 2005-21580 discloses a living body information imaging apparatus which acquires both a photoacoustic image and an ultrasonic image, and facilitates the comprehension of positions within the object by superimposing the two image data or displaying the two image data next to each other.

When imaging and displaying function information, there is a problem in that the contrast inside the region of interest (ROI) becomes insufficient due to the unwanted image components outside the ROI (strong noise and artifacts generated from the boundary with the skin).

For example, strong reflected waves from the skin surface and artifacts based on multiple reflections as unwanted image components among the function information sometimes become a strong signal that is equal to or greater than inside the ROI. When imaging the function information, since pixel values are assigned depending on the input signal, there are cases where the contrast inside the ROI becomes insufficient when the pixel values are decided based on the signal level of the overall image. In addition, when superimposing and displaying image information having two different types of characteristics, such as function information and shape information, it becomes difficult to differentiate the two images if sufficient contrast is not obtained inside the ROI.

Prior art which is related to this field of technology can be found e.g. in document RONALD E KUMON ET AL: "Frequency-Domain Analysis of Photoacoustic Imaging Data From Prostate Adenocarcinoma Tumors in a Murine Model", ULTRASOUND IN MEDICINE AND BIOLOGY, NEW YORK, NY, US, vol. 37, no. 5, 20 January 2011, pages 834-839, in document JP 2012-075464 disclosing photoacoustic diagnostic imaging equipment, image generating method, and program, in document JAEGER M ET AL: "Improved contrast deep optoacoustic imaging using displacement-compensated averaging: breast tumour phantom studies; Improved contrast deep optoacoustic imaging of breast phantoms", PHYSICS IN MEDICINE AND BIOLOGY, INSTITUTE OF PHYSICS PUBLISHING, BRISTOL GB, vol. 56, no. 18, 18 August 2011, pages 5889-5901, in document US 2010/0049044 A1 disclosing combined photoacoustic and ultrasound imaging system, in document US 2004/0220465 A1 disclosing multi-sensor breast tumor detection, in document EP 1 493 380 A1 disclosing method and apparatus for forming an image, in document DE LA ZERDA A ET AL: "Ultrahigh sensitivity carbon nanotube agents for photoacoustic molecular imaging in living mice", NANO LETTERS, AMERICAN CHEMICAL SOCIETY, vol. 10, no. 6, 9 June 2010, pages 2168-2172, in document WO 2012/014390 A2 disclosing photoacoustic diagnostic apparatus, in document US 5,797,397 A disclosing ultrasound imaging system and method using intensity highlighting to facilitate tissue differentiation, in document US 2003/0053668 A1 disclosing device for processing images, in particular medical images, and in document US 5,042,077 A disclosing method of highlighting subtle contrast in graphical images.

SUMMARY OF THE INVENTION

In light of the foregoing problems, this invention provides an object information acquiring apparatus capable of generating a photoacoustic image with sufficient contrast guaranteed within the region of interest.

The present invention in its one aspect provides an object information acquiring apparatus as specified in claims 1 to 4.

The present invention in its another aspect provides an object information acquiring apparatus as specified in claims 5 to 8.

According to the present invention, it is possible to provide an object information acquiring apparatus capable of generating a photoacoustic image with sufficient contrast guaranteed within the region of interest.

Further features of the present invention will become apparent from the following description of exemplary embodiments with reference to the attached drawings.

BRIEF DESCRIPTION OF THE DRAWINGS

FIG. 1 is a diagram showing the overall configuration of the photoacoustic imaging apparatus according to the first embodiment;

FIG. 2 is a diagram showing a modified example of the photoacoustic imaging apparatus according to the first embodiment;

FIG. 3 is a diagram showing a GUI display example of the ROI designation mode according to the first embodiment;

FIG. 4 is a diagram showing a GUI display example of the superimposed image display mode according to the first embodiment;

FIG. 5 is a diagram showing an example of the photoacoustic image of the ROI inner part;

FIG. 6 is a diagram showing an example of the photoacoustic image of the ROI outer part;

FIG. 7 is a diagram showing an example of an ultrasonic image;

FIG. 8 is a diagram showing an example of a superimposed image;

FIG. 9A and 9B are diagrams showing the control flowchart in the first embodiment;

FIG. 10 is a diagram showing the overall configuration of the photoacoustic imaging apparatus according to the second embodiment; and

FIG. 11 is a diagram showing a GUI display example according to the second embodiment.

DESCRIPTION OF THE EMBODIMENTS

Embodiments of the present invention are now explained in detail with reference to the drawings. Note that, as a general rule, the same constituent elements are given the same reference numeral and the redundant explanation thereof is omitted.

(First embodiment)

<System configuration>

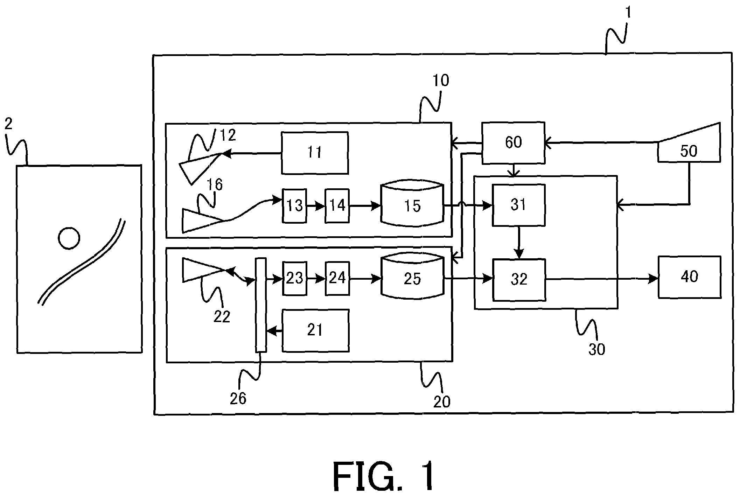

Foremost, the configuration of the photoacoustic imaging apparatus according to the first embodiment is explained with reference to FIG. 1. The photoacoustic imaging apparatus according to the first embodiment of the present invention is an apparatus for imaging information of a living body, which is an object, for the diagnosis of malignant tumors and vascular diseases or the follow-up of chemical treatment. Information of a living body is, for example, the generation source distribution of acoustic waves that were generated based on irradiation of light (hereinafter referred to as "photoacoustic wave"), the initial sound pressure distribution in the living body, or the light energy absorption density distribution that is derived therefrom. In other words, the photoacoustic imaging apparatus according to the first embodiment can also be referred to as an object information acquiring apparatus.

The photoacoustic imaging apparatus according to the first embodiment has a photoacoustic imaging function of emitting measuring light to an object and analyzing the photoacoustic waves to visualize, or image, function information related to the optical characteristics. Moreover, the photoacoustic imaging apparatus also has an ultrasonic imaging function of emitting ultrasonic waves to an object and analyzing the ultrasonic waves (hereinafter referred to as "ultrasonic echo") reflected inside the object to image shape information related to the acoustic characteristics. Moreover, the photoacoustic imaging apparatus also has a function of superimposing and synthesizing (hereinafter simply referred to as "superimposing") the obtained images and displaying the superimposed image. In the ensuing explanation, the image obtained via photoacoustic imaging is referred to as a photoacoustic image and the image obtained via ultrasonic imaging is referred to as an ultrasonic image.

The photoacoustic imaging apparatus 1 according to the first embodiment of the present invention is configured from a photoacoustic image acquiring unit 10, an ultrasonic image acquiring unit 20, an image generating unit 30, an image display unit 40, an operation input unit 50, and a controller unit 60. Note that reference numeral 2 represents a part of the living body as the object. The outline of the method of displaying images is now explained while explaining the respective units configuring the photoacoustic imaging apparatus according to the first embodiment.

<<Photoacoustic image acquiring unit 10>>

The photoacoustic image acquiring unit 10 is a unit for generating photoacoustic images via photoacoustic imaging. For example, it is possible to acquire an image representing the oxygen saturation, which is function information of the living body. The photoacoustic image acquiring unit 10 is configured from a light irradiation control unit 11, a light irradiating unit 12, a photoacoustic signal measuring unit 13, a photoacoustic signal processing unit 14, a photoacoustic image accumulating unit 15, and an ultrasonic probe 16.

The light irradiating unit 12 is a unit for generating near infrared measuring light to be emitting to the living body as the object, and the light irradiation control unit 11 is a unit for controlling the light irradiating unit 12.

It is preferably to generate, from the light irradiating unit 12, light of a specific wavelength that is absorbed by a specific component among the components configuring the living body. Specifically, preferably used is a pulsed light source capable of generating pulsed light in an order of several nano to several hundred nano seconds. While the light source is preferably a light source for generating laser beams, it is also possible to use a light-emitting diode in substitute for the laser beam source. When using a laser, various lasers such as a solid-state laser, gas laser, dye laser or semiconductor laser may be used.

Moreover, the wavelength of the laser beam is preferably in a region of 700 nm to 1100 nm of low absorption within the living body. However, upon obtaining the optical characteristic value distribution of living body tissues relatively near the living body surface, it is also possible to use a wavelength region that is broader than the range of the foregoing wavelength region; for instance, a wavelength region of 400 nm to 1600 nm may also be used. Of the light within the foregoing range, a specific wavelength may be selected based on the component to be measured.

The ultrasonic probe 16 is a unit for detecting the photoacoustic waves that were generated within the living body as the object, and transducing the detected photoacoustic waves into analog electric signals. Since the photoacoustic waves generated from the living body are ultrasonic waves of 100 KHz to 100 MHz, used as the ultrasonic probe 16 is an ultrasonic transducer capable of receiving the foregoing frequency band. Specifically, used is a sensing element utilizing piezoelectric ceramics (PZT) or a microphone-type capacitive sensing element.

Moreover, it is also possible to use a capacitance-type capacitive micromachined ultrasonic transducer (CMUT), magnetic MUT (MMUT) using a magnetic film, or piezoelectric MUT (PMUT) using a piezoelectric thin film.

Note that any kind of sensing element may be used as the ultrasonic probe 16 so as long as it can transduce acoustic wave signals to electric signals.

The analog electric signals transduced by the ultrasonic probe 16 are amplified by the photoacoustic signal measuring unit 13 and converted into digital signals, and then converted into image data by the photoacoustic signal processing unit 14. This image data is the first image in the present invention. The generated image data is stored in the photoacoustic image accumulating unit 15.

<<Ultrasonic image acquiring unit 20>>

The ultrasonic image acquiring unit 20 is a unit for acquiring shape information of the living body via ultrasonic imaging, and generating ultrasonic images. The ultrasonic image may be a B mode image, or an image generated based on the Doppler method or elasticity imaging. The ultrasonic image acquiring unit 20 is configured from an ultrasonic transmission control unit 21, an ultrasonic probe 22, an ultrasonic signal measuring unit 23, a signal processing unit 24, an ultrasonic image accumulating unit 25, and an ultrasonic transmission/reception switch 26.

The ultrasonic probe 22 is a probe that comprises a sensing element as with the ultrasonic probe 16, and can transmit ultrasonic wave beams to the object.

The ultrasonic transmission control unit 21 is a unit for generating signals to be applied to the respective acoustic elements built into the ultrasonic probe 22, and controlling the frequency and sound pressure of the ultrasonic waves to be transmit.

Since the ultrasonic signal measuring unit 23, the signal processing unit 24, and the ultrasonic image accumulating unit 25 are respectively units that perform similar processing as the photoacoustic signal measuring unit 13, the photoacoustic signal processing unit 14, and the photoacoustic image accumulating unit 15, the detailed explanation thereof is omitted. The only difference is whether the signals to be processed are the photoacoustic waves generated inside the object or the ultrasonic echo in which the ultrasonic waves reflected inside the object. Moreover, the image data generated by the ultrasonic image acquiring unit 20 is the second image in the present invention.

The ultrasonic transmission/reception switch 26 is a switch that is controlled by the ultrasonic transmission control unit 21, and is a unit for switching the transmission and reception of ultrasonic waves to and from the ultrasonic probe 22. The ultrasonic transmission control unit 21 transmits the ultrasonic waves in a state of switching the ultrasonic transmission/reception switch 26 to "transmission", and, by switching to "reception" after the lapse of a given time, receives the ultrasonic echo that is returned from inside the object.

<<Image generating unit 30>>

The image generating unit 30 is a unit for performing image processing to the photoacoustic images accumulated in the photoacoustic image accumulating unit 15. Moreover, the image generating unit 30 is also a unit for performing processing of superimposing the processed photoacoustic image and the ultrasonic images accumulated in the ultrasonic image accumulating unit 25, and generating an image to be presented to the user.

The image generating unit 30 is configured from a photoacoustic image processing unit 31, and an image synthesizing unit 32.

The photoacoustic image processing unit 31 is a unit for performing image processing to the photoacoustic images accumulated in the photoacoustic image accumulating unit 15. Details of the processing contents will be explained later.

The image synthesizing unit 32 is a unit for superimposing the photoacoustic image that has been subjected to image processing by the photoacoustic image processing unit 31 and the ultrasonic images accumulated in the ultrasonic image accumulating unit 25 and generating a single sheet of image. In the ensuing explanation, the image generated by the image synthesizing unit 32 is referred to as a superimposed image.

Note that the image generating unit 30 also has a function of generating an operation GUI for performing image processing, and outputting the generated operation GUI to the image display unit 40.

<<Image display unit 40>>

The image display unit 40 is a unit for presenting, to the user, the operation GUI generated by the image generating unit 30. The superimposed image generated by the image generating unit 30 is presented to the user together with the operation GUI.

<<Operation input unit 50>>

The operation input unit 50 is a unit for receiving operation inputs from the user. The unit that is used for operation inputs may be a pointing device such as a mouse or a pen tablet, or a keyboard or the like. Moreover, the operation input unit 50 may also be a device such as a touch panel or a touch screen that is formed integrally with the image display unit 40.

<<Controller unit 60>>

The controller unit 60 is a computer that is configured from a CPU, DRAM, nonvolatile memory, control port and the like which are all not shown. As a result of programs stored in the nonvolatile memory being executed by the CPU, the respective modules of the photoacoustic imaging apparatus 1 are controlled. While the controller unit is a computer in this embodiment, the controller unit may also be specially designed hardware.

<<Arrangement example of ultrasonic probes>>

FIG. 1 shows an example where the ultrasonic probe 16 used by the photoacoustic image acquiring unit 10 and the ultrasonic probe 22 used by the ultrasonic image acquiring unit 20 are mutually independent. Nevertheless, since the ultrasonic probe used for photoacoustic imaging and the ultrasonic probe used for ultrasonic imaging mutually receive ultrasonic waves of the same frequency band, they can be shared.

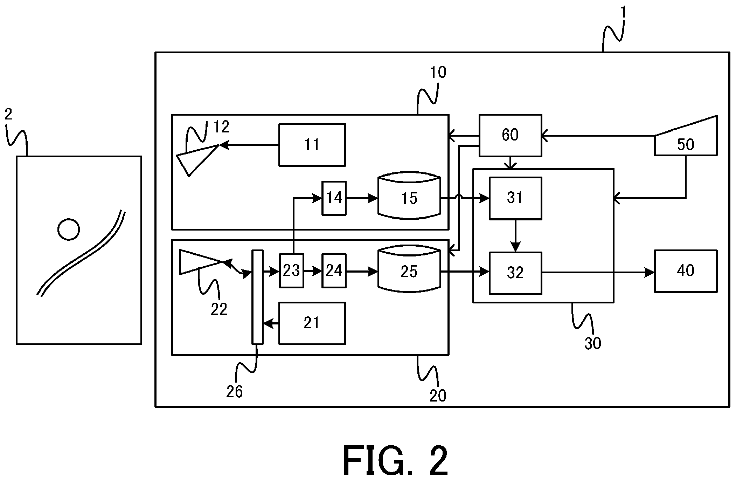

Thus, it is also possible to omit the ultrasonic probe 16, and have the photoacoustic image acquiring unit 10 and the ultrasonic image acquiring unit 20 share the ultrasonic probe 22 based on time sharing control. FIG. 2 is a system configuration diagram showing an example of sharing the ultrasonic probe 22. Note that, since the photoacoustic signal measuring unit 13 can also be shared with the ultrasonic signal measuring unit 23, it is omitted in FIG. 2.

<Operation GUI>

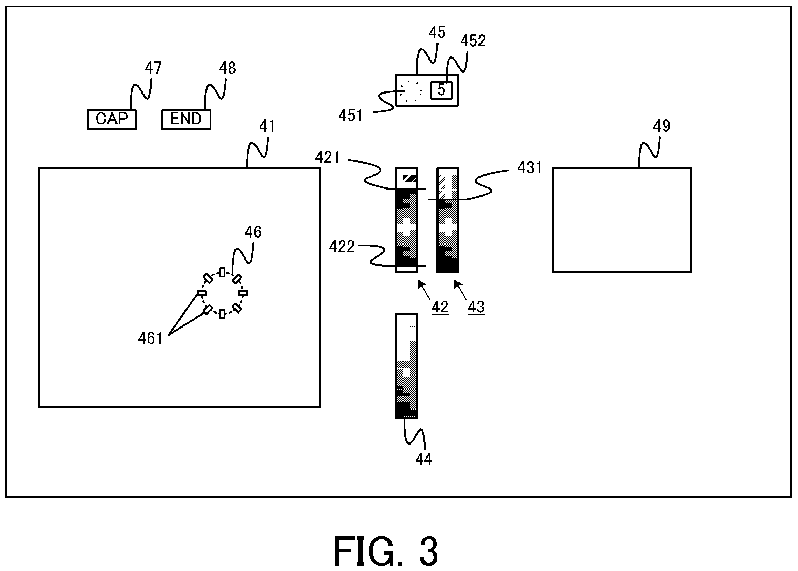

The operation GUI for giving instructions to the photoacoustic imaging apparatus and displaying images is now explained. FIG. 3 shows an example of the operation GUI that is generated by the image generating unit 30 and displayed on the image display unit 40. Here, the respective interfaces configuring the operation GUI are explained.

<<Interface for displaying image>>

The image display region 41 is a region of displaying the photoacoustic image or the superimposed image. In this embodiment, let it be assumed that the ultrasonic image is a B mode image having a width of 40 mm × height (depth) of 30 mm, and 12 bit gradation (4096 gradation) per pixel. Moreover, let it also be assumed that the photoacoustic image is similarly an image having a width of 40 mm × height (depth) of 30 mm, and 12 bit gradation (4096 gradation) per pixel.

Note that, while both the ultrasonic image and the photoacoustic image are gray scale images, the photoacoustic image is subjected to a color display of assigning a different color to each pixel value (that is, brightness value) of the respective pixels in order to increase visibility. For example, the photoacoustic image is displayed by assigning red to the high brightness side, yellowish green to the intermediate value, and blue to the low brightness side. The method of assigning colors will be explained later. Note that, in the ensuing explanation, the photoacoustic image is explained using the term "brightness value" as a gray scale image prior to being colored.

<<Interface for adjusting contrast of photoacoustic image>>

The brightness value designation interface 42 is an interface for performing contrast adjustment of the acquired photoacoustic image. Specifically, the brightness value designation interface 42 is an interface for designating the upper/lower limit of the brightness value upon adjusting the contrast. The lower end represents the lowest brightness, and the upper end represents the highest brightness.

Here, this interface is explained on the assumption that the ROI has been previous designated for the photoacoustic image. The method of designating the ROI will be explained later.

Two types of slide bars are overlapped and displayed on the brightness value designation interface 42. One is the brightness value upper limit slide bar 421, and the other is the brightness value lower limit slide bar 422. On the initial screen of the operation GUI, the respective slide bars are respectively arranged at the position representing the highest brightness and the position representing the lowest brightness among the pixels existing inside the ROI of the photoacoustic image (hereinafter referred to as "pixels inside ROI").

The brightness value of all pixels of the photoacoustic image is reassigned using the range of the brightness value designated with the respective slide bars. For example, considered is a case where the lowest brightness value of the pixels contained in the ROI is n, and the highest brightness value is m. The brightness value lower limit slide bar 422 is disposed at a position representing the brightness value n, and the brightness value upper limit slider bar 421 is disposed at a position representing the brightness value m. In addition, the brightness value of n to m is reassigned to the minimum brightness value to the maximum brightness value. The minimum brightness value or the maximum brightness value is assigned to pixels having a brightness value of n or less or m or more. In other words, image processing in which the contrast inside the ROI is most emphasized is performed on the overall photoacoustic image.

Note that the positions of the respective slide bars can be manually changed to arbitrary positions. When the position of the slide bar is changed, contrast adjustment is once again performed; that is, the brightness value is assigned based on the new position.

Here, the change in contrast when the position of the slide bar is changed is now explained in further detail.

For example, let it be assumed that the user moves the slide bar 421 upward from the initial value based on a drag operation using a mouse. The contrast adjustment is performed, as described above, by performing the processing of reassigning the range of the brightness values designated with the slide bar to the minimum brightness value to the maximum brightness value. Accordingly, processing in which the contrast of the overall image is weakened is performed.

Contrarily, let it be assumed that the slide bar 421 is moved downward from the initial value. Since similar processing is also performed in the foregoing case, image processing in which the contrast of the overall image is emphasized is performed. Since the maximum brightness value is assigned to all pixels having a brightness value that is greater than the brightness value designated with the slide bar 421, the display will become saturated.

Next, considered is a case of moving the slide bar 422 downward from the initial value. In the foregoing case, image processing in which the contrast of the overall image is weakened is performed as with the case of moving the slide bar 421 upward.

Contrarily, let it be assumed that the slide bar 422 is moved upward from the initial value. In the foregoing case, image processing in which the contrast of the overall image is emphasized is performed as with the case of moving the slide bar 421 downward. The minimum brightness value is assigned to all pixels having a brightness value that is smaller than the brightness value designated with the slide bar 422.

As described above, the contrast of the overall image can be adjusted with the two slide bars disposed on the brightness value designation interface 42 so that the visibility inside the ROI becomes highest. The brightness value designation interface 42 generated by the image generating unit 30 and operated by the operation input unit 50 configures the pixel value range designating unit in the present invention.

<<Interface for adjusting opacity of outside ROI of photoacoustic image>>

The ROI outer transparency designation interface 43 is an interface for adjusting the opacity of pixels outside the ROI of the acquired photoacoustic image. With the ROI outer transparency designation interface 43, the lower side represents low opacity (that is, more transparent), and the upper side represents high opacity (that is, more opaque).

One type of slide bar (ROI outer opacity designation slide bar 431) is superimposed and displayed on the ROI outer transparency designation interface 43. The slide bar 431 is a slide bar for designating the opacity of pixels of a region outside the region designated as the ROI (hereinafter referred to as "pixels outside ROI"). On the initial screen, the slide bar 431 is disposed at a value (for example, opacity of 50%) that is set in advance.

The opacity of the pixels outside ROI is set so that it becomes the value indicated with the slide bar. For example, when the slide bar is at a position indicating 50%, image processing of setting the opacity to 50% is performed on the pixels outside ROI of the photoacoustic image.

Note that the slide bar 431 can be used to arbitrarily change the value with a drag operation using a mouse.

Here, considered is a case of dragging the slide bar 431 downward from the initial value. In the foregoing case, image processing of decreasing the opacity outside the ROI is performed. In other words, upon superimposing the images, the transmittance of the pixels outside ROI is increased, and the background image (ultrasonic image in this embodiment) becomes more visible.

Moreover, when the slide bar 431 is dragged upward from the initial value, image processing of increasing the opacity outside the ROI is performed. In other words, upon superimposing the images, the transmittance of the pixels outside ROI is decreased, and the background image becomes less visible.

<<Interface for designating ROI of photoacoustic image>>

The user interface for designating the ROI of the photoacoustic image is now explained.

The ROI designation unit 45 is an interface for designating the ROI of the photoacoustic image. The ROI designation unit 45 is configured from an ROI designation button 451, and an ROI radius display unit 452. By clicking the ROI designation button 451 with a mouse, the mode becomes an ROI designation mode. Moreover, by clicking the ROI designation button 451 once again, the mode becomes a superimposed image display mode.

The ROI designation mode is foremost explained. The ROI designation mode is a mode which enables the operation of designating the ROI. FIG. 3 is a screen display example of the ROI designation mode.

In the ROI designation mode, displayed on the image display region 41 are a photoacoustic image, and an ROI display 46 as a figure for displaying the ROI range. The ROI display 46 is displayed as a circle of a broken line using a color (for example, light purple) that is different from the colors used in the other UI. The ROI display 46 can be moved by dragging it with a mouse.

Moreover, in the ROI designation mode, the ROI radius designation handle 461 is displayed at a total of eight locations; namely, top, bottom, left, right, upper left, lower left, upper right, and lower left of the circle representing the ROI. The user can change the ROI radius by dragging one of the ROI radius designation handles 461 using a mouse.

Here, the ROI radius that is changed based on the drag operation is also simultaneously displayed on the ROI radius display unit 452. Moreover, contrarily, the ROI radius can also be designated by directly inputting the numerical value of the ROI radius into the ROI radius display unit 452. In the foregoing case, the input ROI radius is reflected, and the ROI display 46 is updated. The ROI designation unit 45 and the ROI display 46 which are generated by the image generating unit 30 and operated by the operation input unit 50 configure the region of interest designating unit in the present invention.

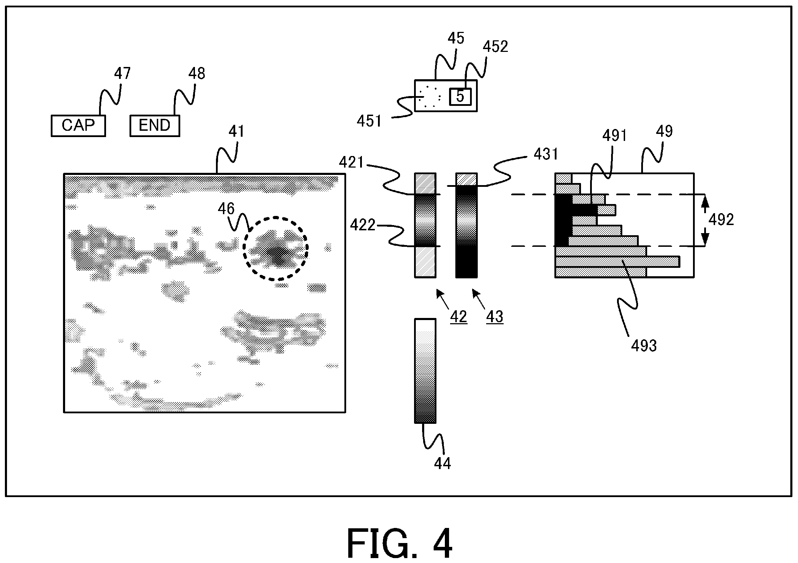

The superimposed image display mode is now explained. The superimposed image display mode is a mode of displaying, on the image display region 41, a superimposed image of the photoacoustic image after image processing; that is, the photoacoustic image after the contrast and opacity have been adjusted, and the ultrasonic image. FIG. 4 is a screen display example in the superimposed image display mode. Note that, for better visibility, FIG. 4 only shows the photoacoustic image. While the circle representing the ROI is displayed in the superimposed image display mode, the ROI radius designation handle 461 is not displayed, and it is not possible to move the ROI or change the radius.

<<Other UI>>

Examples of other UI are now explained with reference to FIG. 4.

Reference numeral 44 shows the region where the scale representing the brightness value of the ultrasonic image is displayed. The maximum brightness value is displayed by being assigned to white, the intermediate value is displayed by being assigned to gray, and the minimum brightness value is displayed by being assigned to black.

Reference numeral 47 shows the image acquiring button for instructing the photoacoustic image acquiring unit 10 and the ultrasonic image acquiring unit 20 to respectively acquire images.

Reference numeral 48 shows the button for instructing the photoacoustic imaging apparatus 1 to end its operation.

Reference numeral 49 shows the histogram display region for displaying the brightness value histogram regarding the pixels inside and outside the ROI of the photoacoustic image. Here, the brightness value histogram of the pixels inside ROI is displayed in black, and the brightness value histogram of the pixels outside ROI is displayed in gray.

<Image processing operation>

Details of the image processing performed by the image generating unit 30 to the photoacoustic image are now explained with reference to FIG. 4.

The image generating unit 30 foremost acquires information regarding the designated ROI, and then generates the ROI inner histogram 491 as the brightness value histogram (frequency distribution) of the pixels inside ROI, and the ROI outer histogram 493 as the brightness value histogram of the pixels outside ROI.

The image generating unit 30 extracts the maximum brightness value and the minimum brightness value of the pixels inside ROI from the ROI inner histogram 491, sets the maximum brightness value as the value of the slide bar 421, and sets the minimum brightness value as the value of the slide bar 422. In the ensuing explanation, the brightness value indicated by the slide bar 421 is represented as $ROI_{max}$, and the brightness value indicated by the slide bar 422 is represented as $ROI_{min}$.

Note that, among the regions represented by the brightness value designation interface 42, a message to the effect that pixels having the brightness value do not exist inside the ROI is displayed in the region above the slide bar 421 and in the region below the slide bar 422. The corresponding regions are filled, for example, with gray.

Subsequently, the brightness value is reassigned using $ROI_{max}$ and $ROI_{min}$ with regard to all pixels in the photoacoustic image. Specifically, the brightness value of pixels having a value of $ROI_{min}$ or less is assigned to the lowest brightness value, the brightness value of pixels having a value of $ROI_{max}$ or more is assigned to the highest brightness value, and the intermediate value is assigned via linear interpolation. Note that the brightness value may also be assigned via methods such as histogram flattening or gamma correction.

Subsequently, assignment of colors for improving the visibility of the image is performed.

When the brightness value is reassigned, the photoacoustic image processing unit 31 replaces the pixels having the maximum brightness value with dark red and the pixels having the lowest brightness value with dark blue relative to the photoacoustic image. With regard to the intermediate brightness value, an arbitrary color display may be assigned.

An example of the color assignment method is shown. Considered is a case where the respective colors of RGB and the color coordinates displaying the opacity $\alpha$ in 8 bits are defined as (R, G, B, $\alpha$), and dark blue, blue, light blue, green, yellow, orange, red, and dark red are assigned in order from the lowest brightness value. The color coordinates of the respective colors can be represented as follows:

dark blue: (0, 0, 128, 255), blue: (0, 0, 255, 255) light blue: (0, 255, 255, 255), green: (0, 255, 0, 255) yellow: (255, 255, 0, 255), orange: (255, 128, 0, 255) red: (255, 0, 0, 255), dark red: (128, 0, 0, 255).

In other words, only the B coordinates change within a range of 128 to 255 between dark blue and blue, only the G coordinates change within a range of 0 to 255 between blue and light blue, and only the B coordinates change within a range of 255 to 0 between light blue and green. Moreover, only the R coordinates change within a range of 0 to 255 between green and yellow, and only the G coordinates change within a range of 255 to 0 among yellow, orange and red. Only the R coordinates change within a range of 255 to 122 between red and dark red. In other words, there are 1280 patterns of color coordinates.

In this embodiment, while the photoacoustic image is of a 12 bit gradation (4096 gradation), since the there are 1280 patterns of the replacement color coordinates, the original brightness value is replaced with 1280 gradation based on contrast adjustment. The value $V_{roi}$ obtained by subjecting the original brightness value $V_{pix}$ to contrast adjustment and being replaced with 1280 gradation will be as shown in Formula 1.

```
(1) When Vpix ≥ ROImax, Vroi = 1280

(2) When ROImin < Vpix < ROImax), Vroi = 1280 × (Vpix - ROImin)

/ (4096 × (ROImax - ROImin))

(3) When Vpix ≤ ROImin, Vroi = 0

...Formula 1 (0 ≤ Vroi ≤ 1280)
```

The method of determining the pixel value of the pixels inside ROI by using the determined $V_{roi}$ is foremost explained. When the determined $V_{roi}$ is replaced with color coordinates, the following is achieved.

```
(1) When 0 ≤ Vroi < 127, (R, G, B, α) = (0, 0, Vroi+128,

255)

(2) When 127 ≤ Vroi < 382, (R, G, B, α) = (0, Vroi-127, 255,

255)

(3) When 382 ≤ Vroi < 637, (R, G, B, α) = (0, 255, 637-Vroi, 255)

(4) When 637 ≤ Vroi < 892, (R, G, B, α) = (Vroi-637, 255, 0, 255)

(5) When 892 ≤ Vroi < 1147, (R, G, B, α) = (0, 1147-Vroi,

255, 255)

(6) When 1147 ≤ Vroi ≤ 1280, (R, G, B, α) = (1402-Vroi, 0,

0, 255)

...Formula 2
```

Accordingly, all pixels inside the ROI can be converted into a color display after adjusting the contrast. Note that the original brightness value and the correspondence of the assigned colors may be displayed, as a color scale, on the brightness value designation interface 42.

The pixel value of the respective pixels of the photoacoustic image outside the ROI is also determined based on the same method as the pixels inside ROI. Nevertheless, since unwanted noise components and artifacts often exist outside the ROI, it is desirable to additionally perform processing of lowering the visibility to the pixels outside ROI.

Thus, in addition to the contrast adjustment that was performed on the pixels inside ROI, visibility is reduced by lowering the opacity for the pixels outside ROI . Here, opacity $\alpha$ is set, and the opacity $\alpha$ is set to all pixels outside the ROI. The opacity $\alpha$ is a value that is designated by the slide bar 431. The initial value is 50% (that is, $\alpha = 128$).

Here, when the designated opacity is $\alpha_{ext}$, the color coordinates of the pixels outside ROI will be as shown in Formula 3. Formula 3 differs only with regard to the designation of opacity in comparison to Formula 2.

(1) When $0 \leq V_{roi} < 127$, $(R, G, B, \alpha) = (0, 0, V_{roi}+128, \alpha_{ext})$

(2) When $127 \leq V_{roi} < 382$, $(R, G, B, \alpha) = (0, V_{roi}-127, 255, \alpha_{ext})$

(3) When $382 \leq V_{roi} < 637$, $(R, G, B, \alpha) = (0, 255, 637-V_{roi}, \alpha_{ext})$

(4) When $637 \leq V_{roi} < 892$, $(R, G, B, \alpha) = (V_{roi}-637, 255, 0, \alpha_{ext})$

(5) When $892 \leq V_{roi} < 1147$, $(R, G, B, \alpha) = (0, 1147-V_{roi}, 255, \alpha_{ext})$

(6) When $1147 \leq V_{roi} \leq 1280$, $(R, G, B, \alpha) = (1402-V_{roi}, 0, 0, \alpha_{ext})$

...Formula 3

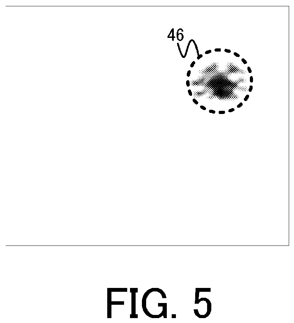

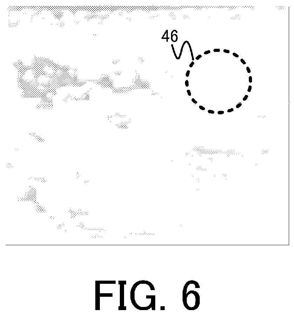

FIG. 5 shows an example of the photoacoustic image of applying Formula 2 and increasing the visibility of the pixels inside ROI. Moreover, FIG. 6 shows an example of the photoacoustic image of applying Formula 3 and reducing the visibility of the pixels outside ROI. In this example, while the images are separately shown in FIG. 5 and FIG. 6 for facilitating the explanation, the photoacoustic image that is generated as a result of the image processing is a single photoacoustic image.

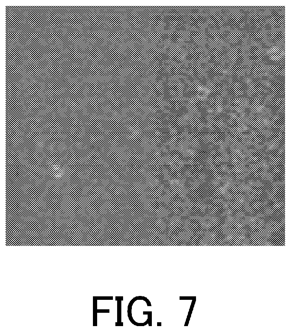

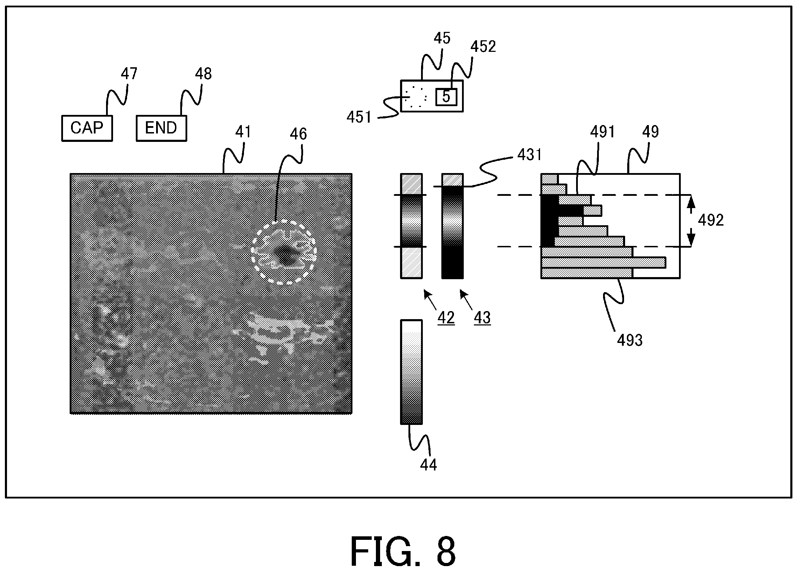

Moreover, FIG. 7 shows an example of the ultrasonic image, and FIG. 8 shows an example of superimposing and displaying the photoacoustic image, which has been subjected to image processing, and the ultrasonic image.

As described above, the photoacoustic imaging apparatus according to the first embodiment can perform image processing for increasing the visibility of the pixels inside ROI based on contrast adjustment, and reducing the visibility of the pixels outside ROI by additionally performing opacity adjustment.

<Processing flowchart>

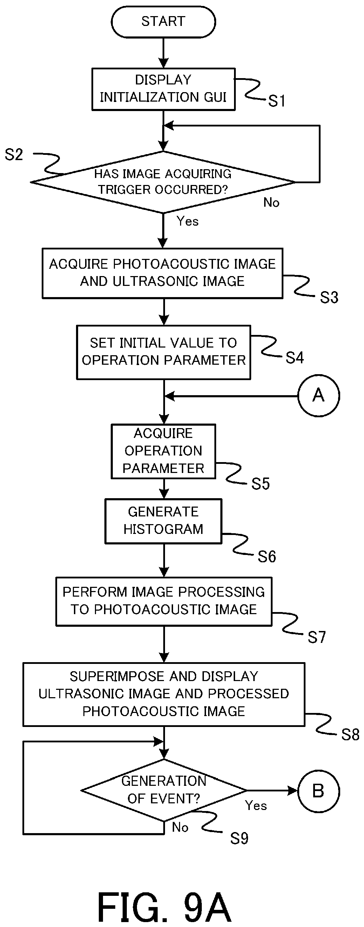

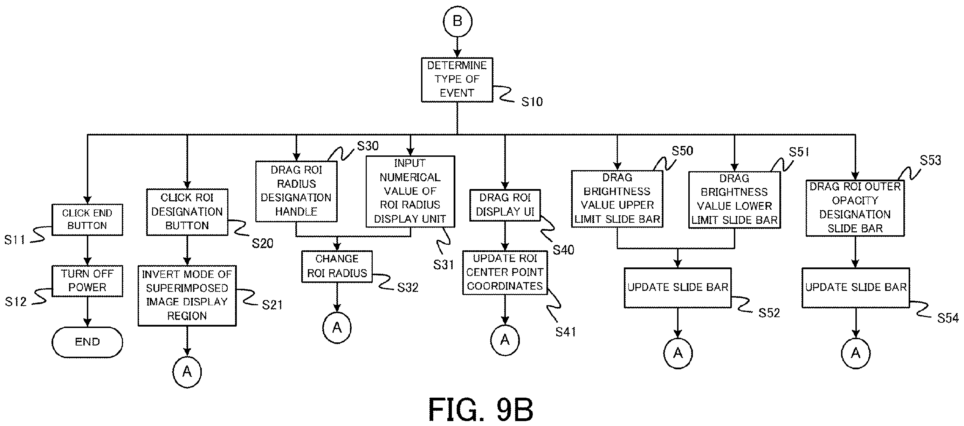

The processing of the photoacoustic imaging apparatus according to the first embodiment generating a superimposed image is now explained with reference to FIG. 9A and FIG. 9B, which are processing flowchart diagrams.

In step S1, after the power of the photoacoustic imaging apparatus 1 is turned ON and the various initializations are performed, the image generating unit 30 displays, on the image display unit 40, the operation GUI shown in FIG. 3.

In step S2, whether the image acquiring button 47 has been clicked is determined. When a click event has occurred, the routine proceeds to step S3, and when a click event has not occurred, the processing waits for an event to occur.

In step S3, the photoacoustic image acquiring unit 10 acquires a photoacoustic image, and the ultrasonic image acquiring unit 20 acquires an ultrasonic image. The photoacoustic image is stored in the photoacoustic image accumulating unit 15, and the ultrasonic image is stored in the ultrasonic image accumulating unit 25.

In step S4, the photoacoustic image processing unit 31 sets the initial value in the operation parameter. An operation parameter is information configured from the current mode (superimposed image display mode or ROI designation mode), center point coordinates of the ROI, and ROI radius. For example, the mode is set as the superimposed image display mode, and the center point coordinates of the ROI are set to the center of the image display region. Moreover, the ROI radius is set to 5 mm.

In step S5, the photoacoustic image processing unit 31 acquires the operation parameter. The mode, center point coordinates of the ROI, and ROI radius are thereby set forth, and the ROI is identified.

In step S6, the photoacoustic image processing unit 31 uses the ROI information identified in step S5 and generates a histogram of the pixels inside ROI and a histogram of the pixels outside ROI. The generated histograms are displayed in the region shown with reference numeral 49.

Moreover, the positions of the slide bars 421, 422 are respectively set to the maximum brightness value and the minimum brightness value of the pixels inside ROI. However, this processing is omitted when the slide bars 421, 422 have been manually moved in the set ROI.

Subsequently, $ROI_{max}$ and $ROI_{min}$ are substituted with the brightness values designated by the slide bars 421, 422. Moreover, $\alpha_{ext}$ is substituted with the opacity designated by the slide bar 431. If the slide bar 431 has never been operated, then the $\alpha_{ext}$ is 128.

In step S7, image processing is performed on the photoacoustic image acquired in step S3. Specifically, the center point coordinates of the ROI and the ROI radius are used to determine whether the pixels configuring the photoacoustic image acquired in step S3 are inside the ROI or outside the ROI, and Formula 1 is used to adjust the brightness values of the pixels, and Formulas 2 and 3 are used to assign colors. Consequently, the photoacoustic image after being subjected to image processing is obtained. The obtained image is temporarily stored.

Moreover, in step S7, the colors assigned to the respective brightness values based on Formulas 1 and 2 are displayed, as a color scale, on the brightness value designation interface 42. The brightness values that does not exist inside the ROI are displayed in gray.

In step S8, the image synthesizing unit 32 superimposed the photoacoustic image, which has been subjected to the image processing in step S7, with the ultrasonic image acquired in step S3, and displays the superimposed image on the image display region 41 together with the ROI display 46. Here, when the mode is the ROI designation mode, the ROI radius designation handle 461 is displayed. When the mode is the superimposed image display mode, the ROI radius designation handle is not displayed.

Step S9 is a step of waiting for the occurrence of an event such as a click or a drag to the respective parts configuring the operation GUI. Once an event occurs, the routine proceeds to step S10 of FIG. 9B.

Step S10 is a step of determining the type of event that occurred. The respective events are now explained.

When the end button 48 is clicked (S11), the routine proceeds to step S12, and the photoacoustic imaging apparatus 1 is shut down to end the processing.

When the ROI designation button 451 is clicked (S20), the routine proceeds to step S21, and the mode is switched by updating the operation parameter indicating the mode. When the current mode is the superimposed image display mode, the mode is switched to the ROI designation mode, and when the current mode is the ROI designation mode, the mode is switched to the superimposed image display mode. Note that, only when the current mode is the ROI designation mode, the dragging of the ROI display 46 and the ROI radius designation handle 461 and the input of numerical values into the ROI radius display unit 452 are enabled. When this processing is ended, the routine proceeds to step S5.

When the ROI radius designation handle 461 is dragged (S30), the routine proceeds to step S32, and the ROI radius is changed. Specifically, the ROI radius is calculated from the handle coordinates upon the completion of dragging and the center point coordinates of the ROI, and the operation parameter indicating the ROI radius is updated.

Moreover, the calculated ROI radius is reflected in the ROI radius display unit 452, and the ROI display 46 is updated. When this processing is ended, the routine proceeds to step S5.

When a numerical value is input into the ROI radius display unit 452 (S31), the processing also proceeds to step S32, and the ROI radius is changed. Specifically, the operation parameter indicating the ROI radius is updated with the input numerical value as the value of the ROI radius. Moreover, the ROI display 46 is updated according to the new ROI radius. When this processing is ended, the routine proceeds to step S5.

When the ROI display 46 is dragged (S40), the routine proceeds to step S41, and the ROI is moved. Specifically, the center point coordinates of the ROI display 46 upon the completion of dragging are acquired, and the acquired center point coordinates are used to update the operation parameter indicating the center point of the ROI. Moreover, the ROI display 46 is updated according to the center point coordinates. When this processing is ended, the routine proceeds to step S5.

When the brightness value upper limit slide bar 421 is dragged (S50), or when the brightness value lower limit slide bar 422 is dragged (S51), the routine proceeds to step S52, and the positions of the respective slide bars are updated. When this processing is ended, the routine proceeds to step S5.

Moreover, when the ROI outer opacity designation slide bar 431 is dragged (S53), the routine proceeds to step S54, and the position of the slide bar 431 is updated. When this processing is ended, the routine proceeds to step S5.

When the respective slide bars are dragged, $ROI_{max}$, $ROI_{min}$, and $\alpha_{ext}$ are re-set in step S6, and the set values are used to perform the image processing in step S7.

In step S8, when an event does not occur or an even other than those described above occurs, the processing is not performed and the routine stands by.

As explained above, in the first embodiment, in a photoacoustic imaging apparatus which superimposes and displays a photoacoustic image and an ultrasonic image, image processing is performed inside the region of interest and outside the region of interest, respectively, by using different image processing parameters. Consequently, it is possible to improve the visibility of signals inside the ROI, and cause the signals (noise, artifacts) outside the ROI to become inconspicuous.

Note that, as a matter of course, the colors to be assigned to the respective pixels of the photoacoustic image may be other than the illustrated colors. For example, the maximum value side may be assigned to white and the minimum value side may be assigned to black to achieve a black and white display, or other color displays may be assigned.

(Second embodiment)

The second embodiment is an embodiment of emitting measuring light of multiple wavelengths to an object, acquiring a plurality of photoacoustic images, and performing image processing to the respective photoacoustic images.

For example, image processing is separately performed on the first photoacoustic image acquired by emitting a laser beam near 750 nm as the first wavelength, and to the second photoacoustic image acquired by emitting a laser beam near 830 nm as the second wavelength, and both of the obtained images are superimposed and displayed. Contents of the image processing performed on the respective images are the same as the first embodiment.

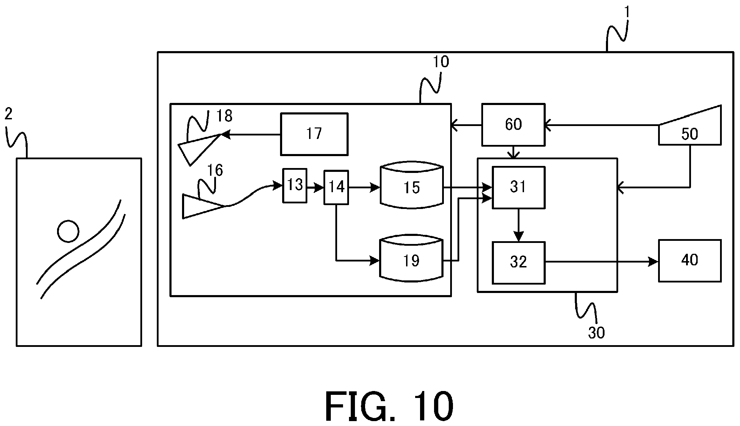

FIG. 10 is a diagram showing the overall configuration of the photoacoustic imaging apparatus according to the second embodiment.

While the light irradiating unit 18 is similar to the light irradiating unit 12 according to the first embodiment, it differs with respect to the point that it can emit laser beams of two different wavelengths. Moreover, while the light irradiation control unit 17 is similar to the light irradiation control unit 11 according to the first embodiment, it differs with respect to the point that it can issue a wavelength switching command to the light irradiating unit 18.

Moreover, the photoacoustic signal processing unit 14 differs from the first embodiment with respect to the point of accumulating the first photoacoustic image obtained by emitting a first wavelength in the first photoacoustic image accumulating unit 15, and accumulating the second photoacoustic image obtained by emitting a second wavelength in the second photoacoustic image accumulating unit 19.

Moreover, the photoacoustic imaging apparatus 1 according to the second embodiment does not includes the ultrasonic image acquiring unit 20. Since the other units are the same as the first embodiment, the explanation thereof is omitted.

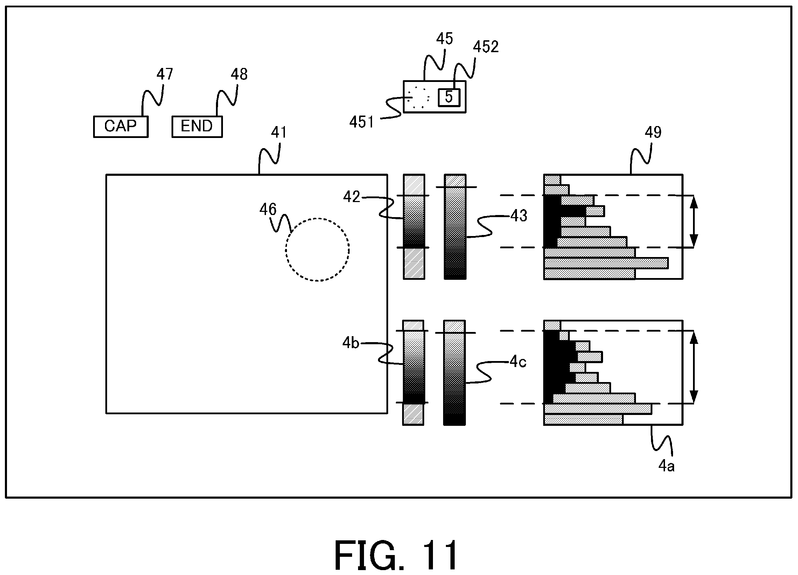

FIG. 11 shows an example of the operation GUI display in the photoacoustic imaging apparatus according to the second embodiment. Here, the differences with the operation GUI display in the first embodiment are explained. The operation GUI display in the second embodiment differs from the first embodiment with respect to the point of comprising two histogram display regions, two brightness value designation interfaces, and two ROI outer transparency designation interfaces, respectively. The respective regions and interfaces correspond to the first photoacoustic image and the second photoacoustic image.

The histogram display region 49 is a histogram display region for displaying the brightness value histogram inside the ROI and outside the ROI of the first photoacoustic image. Moreover, the histogram display region 4a is a histogram display region for displaying the brightness value histogram inside the ROI and outside the ROI of the second photoacoustic image.

Moreover, the brightness value designation interface 42 is an interface for adjusting the brightness value of the first photoacoustic image, and the brightness value designation interface 4b is an interface for adjusting the brightness value of the second photoacoustic image.

Moreover, the ROI outer transparency designation interface 43 is an interface for adjusting the opacity of pixels outside the ROI of the first photoacoustic image, and the ROI outer transparency designation interface 4c is an interface for adjusting the opacity of pixels outside the ROI of the second photoacoustic image. Since the respective operations are the same as the first embodiment, the explanation thereof is omitted.

In the first embodiment, color display was performed by assigning different colors based on the brightness value of the pixels, but in the second embodiment, since the photoacoustic images are superimposed, if the same method is adopted, same colors will be assigned to different images, and differentiation of the images will become difficult.

Thus, in the second embodiment, different tones are used in the first photoacoustic image and the second photoacoustic image for coloring. Specifically, the first photoacoustic image is based on red, and colors are assigned by increasing the lightness on the high brightness side and reducing the lightness on the low brightness side. Moreover, the second photoacoustic image is based on blue, and colors are assigned by increasing the lightness on the high brightness side and reducing the lightness on the low brightness side. It is thereby possible to differentiate the two images.

The method of assigning colors to pixels is now explained.

Foremost, the maximum value $ROI1_{max}$ and the minimum value $ROI1_{min}$ are extracted from the histogram inside the ROI of the first photoacoustic image, and light red (255, 191, 191, 255) is assigned to $ROI1_{max}$, and dark red (128, 0, 0, 255) is assigned to $ROI1_{min}$.

Between dark red and light red, the R coordinates foremost change in a range of 128 to 255, and subsequently the G and B coordinates simultaneously change in a range of 0 to 191. In other words, there are 320 patterns of color coordinates assigned to the first photoacoustic image.

Similarly, the maximum value $ROI2_{max}$ and the minimum value $ROI2_{min}$ are extracted from the histogram inside the ROI of the second photoacoustic image, and light purple (191, 191, 255, 255) is assigned to $ROI2_{max}$, and dark blue (0, 0, 128, 255) is assigned to $ROI2_{min}$.

Between dark blue and light blue, the B coordinates foremost change in a range of 128 to 255, and subsequently the R and G coordinates simultaneously in a range of 0 to 191. In other words, there are similarly 320 patterns of color coordinates assigned to the second photoacoustic image.

In the second embodiment, since there are 320 patterns of the replacement color coordinates, the original brightness value is substituted with 320 gradation based on contrast adjustment. The value $V1_{roi}$ obtained by subjecting the brightness value $V1_{pix}$ of the first photoacoustic image to contrast adjustment and being replaced by 320 gradation will be as shown in Formula 4.

```
(1) When V1pix ≥ ROI1max, V1roi=319

(2) When ROI1min < V1pix < ROI1max, V1roi=319 × (V1pix-

ROI1min)/(4096 × (ROI1max-ROI1min))

(3) When V1pix ≤ ROI1min, V1roi=0

...Formula 4 (0 ≤ V1roi ≤ 319)
```

When $V1_{roi}$ is replaced with color coordinates, the following is achieved.

```
(1) When 0 ≤ V1roi < 128, (R, G, B, α) = (V1roi+128, 0, 0,

αext)

(2) When 128 ≤ V1roi ≤ 319, (R, G, B, α) = (255, V1roi-128,

V1roi-128, αext)

...Formula 5
```

However, when the target pixels are pixels inside ROI, $\alpha_{ext}$ = 255, and, when the target pixels are pixels outside ROI, $\alpha_{ext}$ is set to a value that is designated by the ROI outer opacity designation slide bar displayed on the ROI outer transparency designation interface 43.

Similarly, the value $V2_{roi}$ obtained by subjecting the respective brightness values $V2_{pix}$ of the second photoacoustic image, which is 12 bit gradation (4096 gradation) per pixel, to contrast adjustment will be as shown in Formula 6.

```
(1) When V2pix ≥ ROI2max, V2roi=319

(2) When ROI2min < V2pix < ROI2max, V2roi=319 × (V2pix-

ROI2min)/(4096 × (ROI2max-ROI2min))

(3) When V2pix ≤ ROI2min, V2roi=0

...Formula 6 (0 ≤ V2roi ≤ 319)
```

When $V2_{roi}$ is replaced with color coordinates, the following is achieved.

(1) When $0 \leq V2_{roi} < 128$, $(R, G, B, \alpha) = (0, 0, V2_{roi}+128,$

$\alpha_{ext})$

(2) When $128 \leq V2_{roi} \leq 319$, $(R, G, B, \alpha) = (V2_{roi}-128, V2_{roi}-128,$

$255, \alpha_{ext})$

...Formula 7

However, when the target pixels are pixels inside ROI, $\alpha_{ext} = 255$, and, when the target pixels are pixels outside ROI, $\alpha_{ext}$ is set to a value that is designated by the ROI outer opacity designation slide bar displayed on the ROI outer transparency designation interface 4c.

As described above, by assigning colors using Formulas 4 to 7 to the respective pixels inside the ROI of two types of photoacoustic images, it is possible to perform contrast adjustment and opacity adjustment. Note that, as a matter of course, the method of assigning colors may be other than the illustrated color display assignment.

The photoacoustic imaging apparatus according to the second embodiment superimposes the first and second photoacoustic images which have been subjected to contrast adjustment and opacity adjustment as described above, and displays the superimposed image on the image display region 41.

As explained above, the present invention is not limited to superimposing the photoacoustic image and the ultrasonic image, and can also be applied to cases of superimposing and displaying different photoacoustic images.

With the second embodiment, it is possible to superimpose and display a plurality of photoacoustic images upon individually performing contrast adjustment and opacity adjustment thereto, and thereby improve the visibility of signals inside the ROI and cause the signals (noise, artifacts) outside the ROI to become inconspicuous.

Note that, while the second embodiment illustrated a case of providing two UI each for performing contrast adjustment and opacity adjustment and performing processing to two images, it is also possible to perform contrast adjustment and opacity adjustment on each of three or more images and subsequently superimpose the images.

Note that the explanation of the respective embodiments is an exemplification for explaining the present invention, and the present invention can be implemented by suitably changing or combining the embodiments to the extent that such change or combination will not deviate from the scope of the invention.

For example, while the embodiments explained a case of performing contrast adjustment by designating a range of brightness values in a gray scale image, the input image may also be other than a gray scale image. In the foregoing case, contrast adjustment can also be performed based on the pixel values; that is, the brightness values of the respective colors.

**Claims**

1.   An object information acquiring apparatus, comprising:

a photoacoustic image acquiring unit (10) configured to emit measuring light to an object, receive photoacoustic waves generated in the object, and generate a first image which visualizes information related to optical characteristics within the object based on the photoacoustic waves;
an ultrasonic image acquiring unit (20) configured to transmit ultrasonic waves to the object, receive an ultrasonic echo reflected in the object, and generate a second image which visualizes information related to acoustic characteristics within the object based on the ultrasonic echo;
a region of interest designating unit (45) configured to receive designation of a region of interest with regard to the first image;
an image processing unit (31) configured to perform image processing on the first image inside the designated region of interest and outside the designated region of interest, respectively, using different image processing parameters, one of the image processing parameters used for performing the image processing on the first image outside the designated region of interest being transparency, the image processing performed on the first image inside the designated region of interest including contrast adjustment, and the image processing performed on the first image outside the designated region of interest including the contrast adjustment and transparency adjustment; and

an image synthesizing unit (32) configured to superimpose and synthesize the first image, which has been subjected to the image processing, and the second image;

**characterized by**

a transparency designating unit (43) configured to receive from a user designation of the transparency outside the region of interest of the first image, the transparency being changeable according to the designation of the transparency from the user,

wherein the image processing unit is configured to set the designated transparency only to pixels outside the region of interest of the first image.

2. The object information acquiring apparatus according to claim 1,
wherein the image processing unit is configured to acquire a frequency distribution of brightness values of pixels inside the region of interest of the first image, and perform the contrast adjustment on the first image based on the frequency distribution.

3. The object information acquiring apparatus according to claim 2,
wherein the image processing unit is configured to perform the contrast adjustment on the first image using a maximum value and a minimum value of brightness values of pixels inside the region of interest of the first image.

4. The object information acquiring apparatus according to any of claims 1 to 3, further comprising:

a pixel value range designating unit configured to receive designation of a range of brightness values of pixels to be emphasized in the first image,

wherein the image processing unit is configured to perform the contrast adjustment on the first image using the designated range of brightness values of pixels.

5. An object information acquiring apparatus, comprising:

a photoacoustic image acquiring unit (10) configured to emit measuring light of different wavelengths to an object, receive, for each of the wavelengths, photoacoustic waves generated in the object, and generate, for each of the wavelengths, an image which visualizes information related to optical characteristics within the object based on the photoacoustic waves;

a region of interest designating unit (45) configured to receive designation of a region of interest;

an image processing unit (31) configured to perform image processing on each of plurality of images inside and outside the region of interest, respectively, using different image processing parameters, one of the image processing parameters used for performing the image processing on the image outside the designated region of interest being transparency, the image processing performed on the image inside the designated region of interest including contrast adjustment, the image processing performed on the image outside the designated region of interest including the contrast adjustment and transparency adjustment;

an image synthesizing unit (32) configured to superimpose and synthesize the plurality of images which have been subjected to the image processing, and

a transparency designating unit (43) configured to receive, for each of the plurality of images, from a user designation of the transparency outside the region of interest, the transparency being changeable according to the designation of the transparency from the user,

wherein the image processing unit is configured to set the designated transparency only to pixels outside the region of interest of the respective images.

6. The object information acquiring apparatus according to claim 5,
wherein the image processing unit is configured to acquire, for each of the plurality of images, a frequency distribution of brightness values of pixels inside the region of interest, and perform the contrast adjustment on the respective images based on the frequency distribution.

7. The object information acquiring apparatus according to claim 6,
wherein the image processing unit is configured to perform the contrast adjustment on the respective images using a maximum value and a minimum value of brightness values of pixels inside the region of interest acquired for each of the plurality of image.

8. The object information acquiring apparatus according to any of claims 5 to 7, further comprising:

a pixel value range designating unit configured to receive designation of a range of brightness values of pixels to be emphasized with regard to each of the plurality of images,
wherein the image processing unit is configured to perform contrast adjustment on the respective images using the designated ranges of brightness values of pixels, respectively.

**Patentansprüche**

1. Objektinformationserhaltevorrichtung mit:

   einer fotoakustischen Bilderhalteeinheit (10), die konfiguriert ist, Messlicht auf ein Objekt zu emittieren, fotoakustische Wellen, die in dem Objekt generiert werden, zu empfangen, und ein erstes Bild, das eine Information, die basierend auf den fotoakustischen Wellen auf optische Charakteristiken innerhalb des Objekts bezogen ist, visualisiert, zu erzeugen;
   einer Ultraschallbilderhalteeinheit (20), die konfiguriert ist, Ultraschallwellen auf das Objekt zu transmittieren, ein Ultraschallecho, das in dem Objekt reflektiert wird, zu empfangen, und ein zweites Bild, das eine Information, die basierend auf dem Ultraschallecho auf akustische Charakteristiken innerhalb des Objekts bezogen ist, visualisiert, zu erzeugen;
   einer Interessensgebietbestimmungseinheit (45), die konfiguriert ist, eine Bestimmung eines Interessensgebiets mit Bezug auf das erste Bild zu empfangen;
   einer Bildverarbeitungseinheit (31), die konfiguriert ist, eine Bildverarbeitung auf dem ersten Bild innerhalb des bestimmten Interessensgebiets und außerhalb des bestimmten Interessensgebiets jeweils unter Verwendung von verschiedenen Bildverarbeitungsparametern durchzuführen, wobei einer der Bildverarbeitungsparameter, der zum Durchführen des Bildverarbeitens auf dem ersten Bild außerhalb des bestimmten Interessensgebiets verwendet wird, Transparenz ist, das Bildverarbeiten, das auf dem ersten Bild innerhalb des bestimmten Interessensgebiets durchgeführt wird, Kontrastanpassung enthält, und das bildverarbeiten, das auf dem ersten Bild außerhalb des bestimmten Interessensgebiets durchgeführt wird, das Kontrastanpassen und eine Transparenzanpassung enthält; und
   einer Bildsyntheseeinheit (32), die konfiguriert ist, das erste Bild, das der Bildverarbeitung unterzogen wurde, und das zweite Bild zu überlagern und zu synthetisieren;
   **gekennzeichnet durch**
   eine Transparenzbestimmungseinheit (43), die konfiguriert ist, von einem Nutzer eine Bestimmung der Transparenz außerhalb des Interessensgebiets des ersten Bilds zu empfangen, wobei die Transparenz gemäß der Bestimmung der Transparenz von dem Nutzer änderbar ist;
   wobei die Bildverarbeitungseinheit konfiguriert ist, die bestimmte Transparenz nur für Pixel außerhalb des Interessensgebiets des ersten Bildes einzustellen.

2. Objektinformationserhaltevorrichtung nach Anspruch 1, wobei die Bildverarbeitungseinheit konfiguriert ist, eine Frequenzverteilung von Helligkeitswerten von Pixeln innerhalb des Interessensgebiets des ersten Bildes zu erhalten, und eine Kontrastanpassung auf dem ersten Bild basierend auf der Frequenzverteilung durchzuführen.

3. Objektinformationserhaltevorrichtung nach Anspruch 2, wobei die Bildverarbeitungseinheit konfiguriert ist, die Kontrastanpassung auf dem ersten Bild unter Verwendung eines maximalen Wert und eines minimalen Werts von Helligkeitswerten von Pixeln innerhalb des Interessensgebiets des ersten Bildes durchzuführen.

4. Objektinformationserhaltevorrichtung nach einem der Ansprüche 1 bis 3, ferner mit:

   einer Pixelwertbereichsbestimmungseinheit, die konfiguriert ist, eine Bestimmung eines Bereichs von Helligkeitswerten von Pixeln, die in dem ersten Bild zu betonen sind, zu erhalten,
   wobei die Bildverarbeitungseinheit konfiguriert ist, eine Kontrastanpassung an dem ersten Bild unter Verwendung des bestimmten Bereichs von Helligkeitswerten von Pixeln durchzuführen.

5. Objektinformationserhaltevorrichtung mit:

   einer fotoakustischen Bilderhalteeinheit (10), die konfiguriert ist, Messlicht von verschiedenen Wellenlängen auf ein Objekt zu emittieren, für jede der Wellenlängen fotoakustische Wellen, die in dem Objekt erzeugt werden, zu empfangen, und für jede der Wellenlängen ein Bild, das eine Information, die basierend auf den fotoakustischen Wellen bezogen auf optische Charakteristiken innerhalb des Objektes ist, visualisiert, zu erzeugen;

einer Interessensgebietbestimmungseinheit (45), die konfiguriert ist, eine Bestimmung eines Interessensgebiets zu empfangen;

einer Bildverarbeitungseinheit (31), die konfiguriert ist, eine Bildverarbeitung auf jeder der Vielzahl der Bilder innerhalb und außerhalb des Interessensgebiets jeweils unter Verwendung von verschiedenen Bildverarbeitungsparametern durchzuführen, wobei einer der Bildverarbeitungsparameter, der zum Durchführen des Bildverarbeitens auf dem ersten Bild außerhalb des bestimmten Interessensgebiets verwendet wird, Transparenz ist, das Bildverarbeiten, das auf dem ersten Bild innerhalb des bestimmten Interessensgebiets durchgeführt wird, Kontrastanpassung enthält, und das bildverarbeiten, das auf dem ersten Bild außerhalb des bestimmten Interessensgebiets durchgeführt wird, das Kontrastanpassen und eine Transparenzanpassung enthält;

einer Bildsyntheseeinheit (32), die konfiguriert ist, die Vielzahl der Bilder, die der Bildverarbeitung unterzogen wurden, zu überlagern und synthetisieren, und

einer Transparenzbestimmungseinheit (43), die konfiguriert ist, für jedes der Vielzahl von Bildern von einem Nutzer eine Bestimmung der Transparenz außerhalb des Interessensgebiets zu empfangen, , wobei die Transparenz gemäß der Bestimmung der Transparenz von dem Nutzer änderbar ist;

wobei die Bildverarbeitungseinheit konfiguriert ist, die bestimmte Transparenz nur bei Pixeln außerhalb des Interessensgebiets der jeweiligen Bilder einzustellen.

6. Objektinformationserhaltevorrichtung nach Anspruch 5,
wobei die Bildverarbeitungseinheit konfiguriert ist, für jedes der Vielzahl von Bildern eine Frequenzverteilung von Helligkeitswerten von Pixeln innerhalb des Interessensgebiets zu erhalten und eine Kontrastanpassung auf den jeweiligen Bildern basierend auf der Frequenzverteilung durchzuführen.

7. Objektinformationserhaltevorrichtung nach Anspruch 6,
wobei die Bildverarbeitungseinheit konfiguriert ist, die Kontrastanpassung auf den jeweiligen Bildern unter Verwendung eines Maximalwerts und eines Minimalwerts von Helligkeitswerten von Pixeln innerhalb des Interessensgebiets, die für jedes der Vielzahl der Bilder erhalten werden, durchzuführen.

8. Objektinformationserhaltevorrichtung nach einem der Ansprüche 5 bis 7, ferner mit:

einer Pixelwertbereichsbestimmungeinheit, die konfiguriert ist, eine Bestimmung eines Bereichs von Helligkeitswerten von Pixeln, die zu betonen sind, mit Bezug auf jedes der Vielzahl von Bildern zu erhalten,
wobei die Bildverarbeitungseinheit konfiguriert ist, jeweils eine Kontrastanpassung auf den jeweiligen Bildern unter Verwendung der bestimmten Bereiche von Helligkeitswerten von Pixeln durchzuführen.

## Revendications

1. Appareil d'acquisition d'informations d'objet, comprenant :

une unité d'acquisition d'image photo-acoustique (10) configurée pour émettre une lumière de mesure vers un objet, pour recevoir des ondes photo-acoustiques générées dans l'objet, et pour générer une première image qui visualise des informations se rapportant à des caractéristiques optiques à l'intérieur de l'objet sur la base des ondes photo-acoustiques ;

une unité d'acquisition d'image ultrasonore (20) configurée pour transmettre des ondes ultrasonores vers l'objet, pour recevoir un écho ultrasonore réfléchi dans l'objet, et pour générer une seconde image qui visualise des informations se rapportant à des caractéristiques acoustiques à l'intérieur de l'objet sur la base de l'écho ultrasonore ;

une unité de désignation de région d'intérêt (45) configurée pour recevoir une désignation d'une région d'intérêt par rapport à la première image ;

une unité de traitement d'image (31) configurée pour appliquer un traitement d'image à la première image respectivement à l'intérieur de la région d'intérêt désignée et à l'extérieur de la région d'intérêt désignée, au moyen de paramètres de traitement d'image différents, l'un des paramètres de traitement d'image utilisé pour appliquer le traitement d'image à la première image à l'extérieur de la région d'intérêt désignée étant la transparence, le traitement d'image appliqué à la première image à l'intérieur de la région d'intérêt désignée comprenant un réglage de contraste, et le traitement d'image appliqué à la première image à l'extérieur de la région d'intérêt désignée comprenant le réglage de contraste et le réglage de transparence ; et

une unité de synthèse d'images (32) configurée pour superposer et synthétiser la première image, qui a été soumise au traitement d'image, et la seconde image ;

**caractérisé par**

une unité de désignation de transparence (43) configurée pour recevoir, d'un utilisateur, une désignation de la transparence à l'extérieur de la région d'intérêt de la première image, la transparence pouvant être modifiée conformément à la désignation de la transparence par l'utilisateur,

dans lequel l'unité de traitement d'image est configurée pour ne définir la transparence désignée que par rapport à des pixels à l'extérieur de la région d'intérêt de la première image.

2. Appareil d'acquisition d'informations d'objet selon la revendication 1,

dans lequel l'unité de traitement d'image est configurée pour acquérir une distribution de fréquence de valeurs de luminosité de pixels à l'intérieur de la région d'intérêt de la première image, et pour appliquer un réglage de contraste à la première image sur la base de la distribution de fréquence.

3. Appareil d'acquisition d'informations d'objet selon la revendication 2,

dans lequel l'unité de traitement d'image est configurée pour appliquer le réglage de contraste à la première image au moyen d'une valeur maximale et d'une valeur minimale de valeurs de luminosité de pixels à l'intérieur de la région d'intérêt de la première image.

4. Appareil d'acquisition d'informations d'objet selon l'une quelconque des revendications 1 à 3, comprenant en outre :

une unité de désignation de plage de valeurs de pixels configurée pour recevoir une désignation d'une plage de valeurs de luminosité de pixels à mettre en évidence dans la première image,

dans lequel l'unité de traitement d'image est configurée pour appliquer le réglage de contraste à la première image au moyen de la plage de valeurs de luminosité de pixels désignée.

5. Appareil d'acquisition d'informations d'objet, comprenant :

une unité d'acquisition d'image photo-acoustique (10) configurée pour émettre une lumière de mesure de longueurs d'onde différentes vers un objet, pour recevoir, pour chacune des longueurs d'onde, des ondes photo-acoustiques générées dans l'objet, et pour générer, pour chacune des longueurs d'onde, une image qui visualise des informations se rapportant à des caractéristiques optiques à l'intérieur de l'objet sur la base des ondes photo-acoustiques ;

une unité de désignation de région d'intérêt (45) configurée pour recevoir une désignation d'une région d'intérêt ;

une unité de traitement d'image (31) configurée pour appliquer un traitement d'image à chaque image d'une pluralité d'images respectivement à l'intérieur et à l'extérieur de la région d'intérêt, au moyen de paramètres de traitement d'image différents, l'un des paramètres de traitement d'image utilisé pour appliquer le traitement d'image à la première image à l'extérieur de la région d'intérêt désignée étant la transparence, le traitement d'image appliqué à la première image à l'intérieur de la région d'intérêt désignée comprenant un réglage de contraste, et le traitement d'image appliqué à la première image à l'extérieur de la région d'intérêt désignée comprenant le réglage de contraste et le réglage de transparence ;

une unité de synthèse d'images (32) configurée pour superposer et synthétiser la pluralité d'images qui ont été soumises au traitement d'image, et

une unité de désignation de transparence (43) configurée pour recevoir, d'un utilisateur, pour chaque image de la pluralité d'images, une désignation de la transparence à l'extérieur de la région d'intérêt, la transparence pouvant être modifiée conformément à la désignation de la transparence par l'utilisateur,

dans lequel l'unité de traitement d'image est configurée pour ne définir la transparence désignée que par rapport à des pixels à l'extérieur de la région d'intérêt des images respectives.

6. Appareil d'acquisition d'informations d'objet selon la revendication 5,

dans lequel l'unité de traitement d'image est configurée pour acquérir, pour chaque image de la pluralité d'images, une distribution de fréquence de valeurs de luminosité de pixels à l'intérieur de la région d'intérêt, et pour appliquer le réglage de contraste aux images respectives sur la base de la distribution de fréquence.

7. Appareil d'acquisition d'informations d'objet selon la revendication 6,

dans lequel l'unité de traitement d'image est configurée pour appliquer le réglage de contraste aux images respectives au moyen d'une valeur maximale et d'une valeur minimale de valeurs de luminosité de pixels à l'intérieur de la région d'intérêt acquise pour chaque image de la pluralité d'images.

8. Appareil d'acquisition d'informations d'objet selon l'une quelconque des revendications 5 à 7, comprenant en outre :

une unité de désignation de plage de valeurs de pixels configurée pour recevoir une désignation d'une plage de valeurs de luminosité de pixels à mettre en évidence par rapport à chaque image de la pluralité d'images, dans lequel l'unité de traitement d'image est configurée pour appliquer un réglage de contraste aux images respectives au moyen respectivement des plages de valeurs de pixels désignées.

FIG. 1

FIG. 2

EP 2 749 208 B2

FIG. 3

FIG. 4

FIG. 5

FIG. 6

FIG. 7

FIG. 8

START

DISPLAY
INITIALIZATION GUI — S1

S2 — HAS IMAGE ACQUIRING
TRIGGER OCCURRED?   No

Yes

ACQUIRE PHOTOACOUSTIC IMAGE
AND ULTRASONIC IMAGE — S3

SET INITIAL VALUE TO
OPERATION PARAMETER — S4

Ⓐ

ACQUIRE
OPERATION
PARAMETER — S5

GENERATE
HISTOGRAM — S6

PERFORM IMAGE PROCESSING
TO PHOTOACOUSTIC IMAGE — S7

SUPERIMPOSE AND DISPLAY
ULTRASONIC IMAGE AND PROCESSED
PHOTOACOUSTIC IMAGE — S8

GENERATION
OF EVENT?   Yes   Ⓑ

No — S9

# FIG. 9A

B

DETERMINE
TYPE OF
EVENT — S10

CLICK END
BUTTON
S11

TURN OFF
POWER
S12

END

CLICK ROI
DESIGNATION
BUTTON
S20

INVERT MODE OF
SUPERIMPOSED
IMAGE DISPLAY
REGION — S21

A

DRAG ROI
RADIUS
DESIGNATION
HANDLE — S30

CHANGE
ROI RADIUS — S32

A

INPUT
NUMERICAL
VALUE OF
ROI RADIUS
DISPLAY UNIT
S31

DRAG ROI
DISPLAY UI
S40

UPDATE ROI
CENTER POINT
COORDINATES

A — S41

DRAG
BRIGHTNESS
VALUE UPPER
LIMIT SLIDE BAR — S50

DRAG
BRIGHTNESS
VALUE LOWER
LIMIT SLIDE BAR — S51

UPDATE SLIDE BAR
S52

A

DRAG ROI OUTER
OPACITY
DESIGNATION
SLIDE BAR — S53

UPDATE SLIDE BAR
S54

A

FIG. 9B

FIG. 10

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005021580 A
- JP 2012075464 A
- US 20100049044 A1
- US 20040220465 A1
- EP 1493380 A1
- WO 2012014390 A2
- US 5797397 A
- US 20030053668 A1
- US 5042077 A

### Non-patent literature cited in the description

- **RONALD E KUMON et al.** Frequency-Domain Analysis of Photoacoustic Imaging Data From Prostate Adenocarcinoma Tumors in a Murine Model. *ULTRASOUND IN MEDICINE AND BIOLOGY, NEW YORK, NY, US,* 20 January 2011, vol. 37 (5), 834-839
- Improved contrast deep optoacoustic imaging using displacement-compensated averaging: breast tumour phantom studies; Improved contrast deep optoacoustic imaging of breast phantoms. **JAEGER M et al.** PHYSICS IN MEDICINE AND BIOLOGY. INSTITUTE OF PHYSICS PUBLISHING, 18 August 2011, vol. 56, 5889-5901
- Ultrahigh sensitivity carbon nanotube agents for photoacoustic molecular imaging in living mice. **DE LA ZERDA A et al.** NANO LETTERS. AMERICAN CHEMICAL SOCIETY, 09 June 2010, vol. 10, 2168-2172